# EUROPÄISCHE PATENTSCHRIFT

(11) **EP 1 874 877 B1**
(45) Veröffentlichungstag und Bekanntmachung des Hinweises auf die Patenterteilung: **28.04.2010**
(21) Anmeldenummer: 06723693.5
(22) Anmeldetag: 24.03.2006
(51) Int. Cl.: C09D 5/36, C09D 11/02, C09D 17/00

(54) **PULVERFÖRMIGE PERLGLANZZUSAMMENSETZUNG, VERFAHREN ZU DEREN HERSTELLUNG UND DEREN VERWENDUNG**
POWDERY PEARLY LUSTRE COMPOSITION, METHOD FOR THE PRODUCTION THEREOF AND USE THEREOF
COMPOSITION SOUS FORME DE POUDRE CONFÉRANT UN EFFET NACRÉ ET PROCÉDÉ DE PRODUCTION ET D'UTILISATION DE LADITE COMPOSITION

(30) Priorität: 25.04.2005 DE 102005019431
(43) Veröffentlichungstag der Anmeldung: 09.01.2008
(73) Patentinhaber: Eckart GmbH, 91235 Hartenstein (DE)
(72) Erfinder: BECKER, Michael, 91207 Lauf (DE); OPPER-LINNERT, Helga, 91284 Neuhaus (DE)
(74) Vertreter: Walcher, Armin
(86) Internationale Anmeldenummer: PCT/EP2006/002712
(87) Internationale Veröffentlichungsnummer: WO 2006/114171

(56) Entgegenhaltungen:
- DE-A1- 19 708 167
- DE-A1- 19 826 624
- DE-A1- 19 955 477

## Beschreibung

Die Erfindung betrifft eine pulverförmige Perlglanzpigment-haltige Pigmentzusammensetzung, ein Verfahren zur Herstellung der pulverförmigen Pigmentzusammensetzung und deren Verwendung.

Perlglanzpigmente werden gewöhnlicherweise als Pulver angeboten. Als solches jedoch hat das Pigment stark staubende Eigenschaften, die bei der Einarbeitung in eine Druckfarbe oder in einen Lack stark störend wirken.

Die Staubeigenschaften des Perlglanzpigrnentpulvers können verringert werden, wenn die Perlglanzpigmente als Pigmentpräparation vorliegen. Die Pigmentpräparationen enthalten i.d.R. Zusätze an Bindemittel und häufig geringe Lösemittelmengen.

Die EP 0 994 166 B1 offenbart eine nichtstaubende, homogene Pigmentpräparation mit mindestens 50 Gew.-% eines oder mehrerer Effektpigmente, 0,1 - 50 Gew-% eines Gemisches aus Weichmacher/Nitrocellulose, wobei das Verhältnis Weichmacher/Nitrocellulose 1:1 bis 1:9 beträgt, und 0 - 49,9 Gew.-% eines Lösemittels oder Lösemittelgemisches. Diese Pigmentpräparation ist jedoch bei sehr niedrigen Weichmacher/Nitrocellulose-Könzentrationen nach wie vor staubend und bei höheren Weichmacher/Nitrocellulose-Konzentrationen nicht kompatibel mit wasserbasierenden Applikationssystemen.

Die DE 198 26 624 A1 offenbart eine nichtstaubende, homogene Pigmentpräparation mit mindestens 40 Gew.-% eines oder mehrer Effektpigmente und.0,5 -60 Gew-% eines Polyalkylenglykols und/oder eines oder mehrere Polyalkylenglykol-Derivate. Gemäß der DE 198 26 624 A1 enthält die Pigmentpräparation vorzugsweise erhebliche Mengen an Bindemittel. Eine solche Pigmentpräparation kann mithin nur in Applikationssysteme eingearbeitet werden, bei denen das Bindemittel des Applikationssystems und der Pigmentpräparation aufeinander abgestimmt sind.

Die DE 102 28 199 A1 offenbart Pigmentzubereitungen, bei denen neben anionischen oberflächenaktiven Additiven auch nichtionische oberflächenaktive Additive auf Basis von Polyethern enthalten sind.

Die DE 102 27 657 A1 offenbart feste Pigmentzubereitungen die 60 bis 90 Gew.-% Pigment, und 10 bis 40 Gew.-% mindestens eines oberflächenaktiven Additivs aus der Gruppe der nichtionischen Polyether und ihrer sauren Phosphorsäure-, Phosphonsäure-, Schwefelsäure- und/oder Sulfonsäureester und deren Salzen enthalten. Diese Pigmentzubereitungen sind nur zur Einfärbung von Kunststoffen geeignet.

Die DE 199 55 477 A1 betrifft eine nicht staubende homogene Pigmentpräparation, die ≥ 70 Gew.-% eines oder mehrerer Effektpigmente. 0,1 bis 30 Gew.-% eines organischen Lösemittels oder Lösemittelgemisches mit einer Verdunstungszahl (VZ) von 10 bis 100 und einer Oberflächenspannung (ST) von ≤ 35 mN/m und gegebenenfalls 0 bis 5 Gew.-% einer oberflächenaktiven Substanz enthält.

Die DE 197 08 167 A1 betrifft eine nicht staubende homogene Pigmentpräparation, die 40 bis 60 Gew.-% ein oder mehrerer Perlglanzpigmente, 0,1 bis 5 Gew.-% an oberflächenaktiven Substanzen, 0,5 bis 20 Gew.% an organischen Polymeren, 1 bis 40 Gew.-% eines organischen Lösungsmittels oder Lösungsmittelgemisches, 0 bis 50 Gew.-% Wasser und gegebenenfalls 0 bis 10 Gew.-% eines pH-Reglers enthält.

Bei diesen bekannten Pigmentpräparationen ist nachteilig, daß diese nach dem Verdrucken in Form einer wässrigen Druckfarbe nach dem Trocknen kein Druckerzeugnis mit ausreichender Wasserbeständigkeit ergeben.

Aufgabe der vorliegenden Erfindung ist es, eine konzentrierte Darreichungsform für Perlglanzpigmente zu finden, die nichtstaubend und rieselfähig ist und eine maximale Kompatibilität mit dem Applikationssystem aufweist. Weiterhin sollte die Darreichungsform für Perlglanzpigmente in Form von wässrigen Druckfarben verdruckt werden können, wobei die erhaltenen Druckerzeugnisse über eine sehr gute Wasserbeständigkeit verfügen sollen.

Diese Aufgabe wurde durch Bereitstellung einer nichtstaubenden pulverförmigen, im Wesentlichen bindemittelfreien Pigmentzusammensetzung gelöst, wobei die Pigmentzusammensetzung folgende Bestandteile enthält:
- wenigstens 60 Gew.-% mindestens eines Periglanzpigmentes
- 1 - 15 Gew.-% nichtionisches monomeres Netzmittel und/oder nichtionisches Netzmittel auf Polysiloxan-Basis
- 1 bis 39 Gew.-% Lösemittel oder Lösemittelgemisch,
wobei sich die Summe der Einzelkomponenten zu 100 Ges.-% addiert, wobei des nichtionische Netzmittel OH-Gruppen trägt und eine Hydroxylzahl von 30 bis 150 mg KOH/g Netzmittel aufweist.

Bevorzugte Ausführungsformen sind in den Unteransprüchen angegeben.

Erfindungsgemäß trägt das nichtionische Netzmittel OH-Gruppen. Vorzugsweise weist das OH-Gruppen tragende nichtionische Netzmittel eine Hydroxylzahl von 50 bis 120 mg KOH/g Netzmittel auf. Eine Hydroxylzahl von 75 bis 110 hat sich als sehr geeignet erwiesen. Die Hydroxylzahl lässt sich hierbei gemäß DIN EN ISO 4629 bestimmen.

Gemäß einer erfindungsgemäßen Ausgestaltung enthält die Pigmentzusammensetzung zusätzlich 1 bis 10 Gew.-%, vorzugsweise 2 bis 7 Gew.-% Entschäumer. Die Verwendung von Entschäumer kann von Vorteil sein, wenn das Applikationsmedium, in das die erfindungsgemäße Pigmentzusammensetzung eingearbeitet wird, zum Schäumen oder zur Blasenbildung neigt.

Als Perlglanzpigment kann jedes Perlglanzpigment, insbesondere die herkömmlicherweise bekannten und im Handel erhältlichen Perlglanzpigmente, verwendet werden.

Vorzugsweise werden die Perlglanzpigmente aus der Gruppe, die aus mit hochbrechendem/en Metalloxid(en) beschichteten Glimmer, mit hochbrechendem/en Metalloxid(en) beschichteten Glasplättchen, mit hochbrechendem/en Metalloxid(en) beschichteten SiO₂-Flakes, mit hochbrechendem/en Metalloxid(en) beschichteten Al₂O₃-Flakes, TiO₂-Flakes, Eisenoxidflakes und deren Mischungen, besteht, ausgewählt.

Perlglanzpigmente bestehen generell aus einem niedrigbrechenden, plättchenförmigen Substrat und einem hochbrechendem Metalloxid oder Metallsulfid.

Als Perlglanzsubstrate werden vorzugsweise Glimmer, Kaolin. Glasplättchen, synthetische SiO₂-Plättchen, wie beispielsweise solche, die gemäß der Lehre der EP 1 266 977 A2 erhältlich sind, oder synthetische Aluminiumoxidplättchen verstanden.

Als hochbrechende Metalloxide bzw. Metalloxidhydrate oder Metallsuboxide werden bevorzugt jene aus der Gruppe, bestehend aus Titan, Eisen, Zirkonium, Zink, Zinn, Nickel, Cobalt und/oder Chrom, verwendet.

Der Brechungsindex der Metalloxidschicht ist, um einen guten Periglanzeffekt zu ergeben, vorzugsweise größer als 1,8, bevorzugt größer als 2,2, noch weiter bevorzugt größer als 2,4 und besonders bevorzugt 2,5 oder größer.

Weiterhin können als Perlglanzpigmente auch reine Titandioxid- und/oder reine Eisenoxidplättchen verwendet werden.

Vorzugsweise werden folgende Perlglanzpigmente verwendet:
- Titandioxid und/oder Eisenoxid beschichtete Glimmerplättchen,
- Titandioxid und/oder Eisenoxid beschichtete Glasplättchen,
- Titandioxid und/oder Eisenoxid beschichtete Aluminiumoxidplättchen,
- Titandioxid und/oder Eisenoxid beschichtete SiO₂-Plättchen,
- Bismutoxychloridplättchen,
sowie Mischungen all dieser Perlglanzpigmente.

Bevorzugt finden mit Titandioxid und mit Eisenoxid (Fe₂O₃ und/oder Fe₃O₄) beschichtete Substrate Verwendung. Mit Titandioxid und/oder Eisenoxid beschichtete Glimmerpigmente sind beispielsweise unter dem Namen PHOENIX® (Fa. Eckart GmbH & Co. KG, Fürth, Deutschland) oder unter dem Namen Iriodin® (Fa. Merck GmbH & Co. KG), Darmstadt) im Handel erhältlich. Mit Titandioxid und/oder Fe₂O₃ beschichtete Al₂O₃-Flakes werden unter dem Handelsnamen Xirallic® und entsprechend beschichtete SiO₂-Flakes unter dem Handelsnamen Colorstream® von der Fa. Merck GmbH & Co. KG, Darmstadt, Deutschland, angeboten. Auch mehrschichtige Interferenzpigmente, wie sie z.B. in der DE 19618569 beschrieben sind, bestehend aus einem Trägermaterial, welches mit alternierenden Schichten von Metalloxiden mit niedriger und hoher Brechzahl beschichtet ist, können erfindungsgemäß verwendet werden.

Derartige Perlglanzpigmente werden für Außenanwendungen mit weiteren Schutzschichten versehen, die ihnen Wetterstabilität verleihen. Derartige Schutzschichten enthalten Metalloxide und/oder Metallhydroxide, die vorzugsweise aus der Gruppe, bestehend aus SiO₂, Al₂O₃, Ceroxid, Zirkoniumoxid und Mischungen davon, ausgewählt werden.

Plättchenförmig sind die Perlglanzpigmente, die einen Formfaktor (Verhältnis des Mittelwertes der Längsausdehnung zur mittleren Dicke) von 3 bis 2.000, bevorzugt von 5 bis 500 und ganz besonders bevorzugt von 10 bis 350 haben.

Die Größenverhältnisse der plättchenförmigen Perlglanzpigmente werden charakterisiert durch den Mittelwert (d₅₀-Wert) der Summenverteilungskurve, wie sie üblicherweise mit Laserbeugungsmethoden (z.B. Malvem von der Firma Malvem, Deutschland) gemessen werden. Hierbei sind bevorzugt Größen mit einem d₅₀ von 1 bis 500 µm, besonders bevorzugt von 3 bis 100 µm und ganz besonders bevorzugt von 5 bis 35 µm.

Ferner werden erfindungsgemäß unter dem Begriff "Perlglanzpigmente" auch Pigmente mit einer opalartigen Struktur, wie sie in den Patentanmeldungen DE 102 28 228 A1 oder WO 01/88044 A1 offenbart sind, oder einer inversen opalartigen Struktur, wie aus der DE 102 45 848 A1 bekannt sind, verstanden. Derartige Pigmente weisen im Gegensatz zu den üblichen Perlglanzpigmenten nicht notwendigerweise eine Plättchenstruktur auf.

Vorzugsweise ist das Lösemittel Wasser. Bei Verwendung eines Lösemittelgemischs ist bevorzugt, daß dies im Wesentlichen aus Wasser besteht. Vorzugsweise enthält ein solches Lösemittelgemisch wenigstens 70 Vol.-% Wasser, weiter bevorzugt wenigstens 80 Vol.-%, noch weiter bevorzugt wenigstens 90 Vol.-%. Gemäß einer weiteren Variante enthält das Lösemittelgemisch wenigstens 95 Vol.% Wasser.

Gemäß einem Aspekt der vorliegenden Erfindung enthält die Pigmentzusammensetzung weniger als 1 Gew.-%, vorzugsweise weniger als 0,5 Gew.-% Bindemittel. Bevorzugt ist die Pigmentzusammensetzung bindemittelfrei.

Ein großer Vorteil der erfindungsgemäßen Pigmentzusammensetzung ist der äußerst geringe Gehalt an Bindemittel oder die vollständige Abwesenheit von Bindemittel.

Die erfindungsgemäße Pigmentzusammensetzung kann mithin äußerst vorteilhaft in die verschiedensten Applikationssysteme eingearbeitet werden, ohne daß Komplikationen aufgrund von Inkompatibilitäten verschiedener Bindemittel zu erwarten sind.

Gemäß einer bevorzugten Weiterbildung der Erfindung weist das monomere Netzmittel folgende Struktur (I) auf: 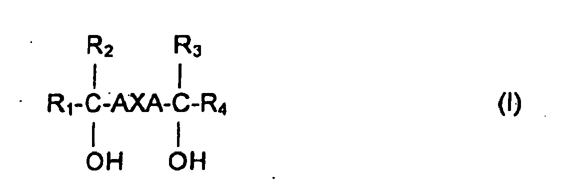 wobei R₁, R₂, R₃ und R₄ unabhängig voneinander Wasserstoff, ein geradkettiger oder verzweigtkettiger Alkylrest mit jeweils 1 bis 10 Kohlenstoffatomen, der jeweils OH-Gruppen aufweisen kann, ein Arylrest mit 1 bis 12 Kohlenstoffatomen, wobei der Arylrest mit Alkylresten mit 1 bis 10 Kohlenstoffatomen substituiert sein kann, wobei Alkyl- und/oder Arylrest jeweils OH-Gruppen aufweisen können, sein können und X für eine Einfachbindung, Doppelbindung oder Dreifachbindung steht, wobei A für CH₂ steht, wenn X eine Einfachbindung ist, A für CH steht, wenn X eine Doppelbindung ist, und A für C steht, wenn X eine Dreifachbindung ist.

Vorzugsweise sind R₁, R₂ R₃ und R₄ unabhängig voneinander Methyl, Ethyl, n-Propyl, Isopropyl, n-Butyl, Isobutyl, tert-Butyl. Weiter bevorzugt sind R₁, und R₄ jeweils tert-Butyl und R₂ und R₃. Vorzugsweise sind R₁, R₂, R₃ und R₄ jeweils ein Methylrest.

Es hat sich überraschend gezeigt, daß bei Verwendung von nichtionischem Netzmittel gemäß Struktur (I) die Perlglanzpigmente, welche flächige Pigmente mit einer Längserstreckung von bevorzugt etwa 1 µm bis etwa 200 µm, vorzugsweise von etwa 10 bis 150 µm, sind und eine Dicke von vorzugweise etwa 0,3 µm bis 10 µm, vorzugsweise von etwa 1 µm bis etwa 5 µm, aufweisen, ein hervorragendes Orientierungsverhalten in einer aufgebrachten Farbe, Druckfarbe oder einem Lack zeigen. Dieses Orientierungsverhalten ist für den visuellen Eindruck eines Betrachters sehr wesentlich, da es die optischen Eigenschaften des Farb- oder Lackfilmes sehr vorteilhaft beeinflußt. Die Perlglanzpigmente der erfindungsgemäßen Perlglanzpigmentzusammensetzung orientieren sich dabei nach der Applikation im Wesentlichen parallel zur Substratoberfläche. Eine Substratoberfläche kann beispielsweise ein Bedruckstoff wie Papier oder eine zu lackierende Fahrzeugkarosserie sein. Einhergehend mit der guten Orientierung der Pigmente wird nach der Applikation ein sehr guter Glanz erhalten.

Die der vorliegenden Erfindung zugrundeliegende Aufgabe wird auch durch ein Verfahren zur Herstellung einer nichtstaubenden pulverförmigen, im Wesentlichen bindemittelfreien Pigmentzusammensetzung nach einem der Ansprüche 1 bis 9, gelöst, wobei die einzelnen Komponenten, vorzugsweise schonend, miteinander homogen vermischt werden.

Die der vorliegenden Erfindung zugrundeliegende Aufgabe wird auch durch eine Verwendung der nichtstaubenden pulverförmigen, im Wesentlichen bindemittelfreien Pigmentzusammensetzung nach einem der Ansprüche 1 bis 9 in Farben, insbesondere Druckfarben, Lacken und kosmetischen Formulierungen gelöst.

Es hat sich überraschend gezeigt, daß die nach einem Verdrucken von Druckfarben erhaltenen Drucke oder nach einem Aufbringen von Lacken erhaltenen Beschichtungen gegenüber wässrigen Medien oder Wasser sehr stabil bzw. beständig sind. Die Beständigkeit ist dabei auf die erfindungsgemäße Pigmentzusammensetzung in den Farben, Druckfarben und Lacken zurückzuführen.

Die Beständigkeit oder Widerstandsfähigkeit der Drucke gegenüber Wasser, die auch als "Wasser-Echheit" bezeichnet wird, wird dabei gemäß DIN 16524 (November 1965) bestimmt.

Unter "pulverförmig" wird im Sinne der Erfindung verstanden, daß die Perlglanzpigmente auch in Aggregaten, beispielsweise granulatförmig, vorliegen können. Die Perlglanzpigmente liegen in der erfindungsgemäßen Pigmentzusammensetzung jedoch nicht in kompaktierter Form wie Pellets, Würstchen, Kugeln, Brickets oder Tabletten vor, wie diese beispielsweise aus der EP 97 104 851.7 bekannt sind, die in Gegenwart von Bindemittel kompaktierte Perlglanzpigment-Zubereitungen offenbart. Dennoch sind alle Vorteile der Pigmentpräparationen in kompaktierten Formen wie beispielsweise Staubfreiheit, Volumenschwund gegenüber dem Pulver, nicht schmierende Eigenschaften usw. bei den erfindungsgemäßen Pigmentzusammensetzungen gegeben.

Die erfindungsgemäße Pigmentzusammensetzung ist rieselfähig und mithin einfach zu dosieren. Äußerst vorteilhaft lässt sich die erfindungsgemäße Pigmentzusammensetzung einfach in Applikationssysteme von Farben, Druckfarben und Lacken einarbeiten, so daß die Perlglanzpigmente homogen in dem Applikationssystem dispergiert vorliegen.

Die erfindungsgemäßen Pigmentzusammensetzungen weisen eine pulverförmige bis granulatförmige, nichtstaubende Konsistenz auf. Die erfindungsgemäßen Pigmentzusammensetzungen sind üblicherweise unregelmäßig geformt.

Die Pulverform ist vorteilhaft gegenüber der Pastenform, die bei zu geringem Pigmentanteil bzw. zu hohem Lösemittelanteil entsteht, da Pulver während der Einarbeitung der Pigmente in Applikationssysteme nicht schmiert. Dies hat für den Anwender Vorteile, da eine bessere Dosierbarkeit zum Applikationssystem gegeben ist und die Vorrichtungen zur Einarbeitung von Pigmenten in ein Applikationssystem von beispielsweise Farben, insbesondere Druckfarben, oder Lacken weniger verschmutzt werden. Da die erfindungsgemäßen Perlglanzpigmentzusammensetzungen im Unterschied zu reinen Periglanzpigmentpulvem, die i.d.R. stark staubende Eigenschaften aufweisen, nichtstaubend sind, sind sie in produktionstechnischer und arbeitssicherheitstechnischer Hinsicht leichter handhabbar.

Die erfindungsgemäße Pigmentzusammensetzung enthält vorzugsweise 60 bis 95 Gew.-%, bevorzugt 70 bis 90 Gew.-% und besonders bevorzugt 71 bis 85 Gew.-% Perlglanzpigmente.

Unterhalb von 60 Gew.-% kann es sein, daß die Pigmentzusammensetzung nicht ausreichend konzentriert ist und eine pastenförmige Konsistenz annimmt. Oberhalb von 95 Gew.-% kann es sein, daß die Pigmentzusammensetzung zu stauben beginnt.

Das nichtionische Netzmittel ermöglicht eine sehr gute Einarbeitbarkeit des Pigmentpulvers in das Applikationssystem bzw. Anwendungsmedium. Anionische Netzmittel zeigen dagegen oft eine starke Schaumentwicklung bei der Einarbeitung einer Pigmentpräparation in ein Applikationssystem. Zudem wird nur eine geringe Wasserfestigkeit bei dem Druckerzeugnis bzw. lackierten Gegenstand erhalten.

Überraschenderweise zeigte es sich, dass die unter Verwendung der erfindungsgemäßen Pigmentzusammensetzungen hergestellten Druckerzeugnisse oder Lackierungen eine sehr gute Wasserbeständigkeit bzw. Wasserechtheit besitzen.

Druckfarbenfilme ohne Überlackierung müssen nach der Trocknung bei vielen Applikationen (wie z.B. Tapetendruck, Geschenkpapier, Servietten, Faltschachteln) eine gute Wasserbeständigkeit aufweisen. Das bedruckte Objekt kommt hier erneut mit Wasser in Kontakt und die Druckfarbe oder deren Bestandteile dürfen sich dabei nicht mehr ablösen.

Geeignete nichtionische Netzmittel sind beispielsweise Disperbyk-107 und Disperbyk-108 der Fa. Byk-Chemie GmbH (Postfach 100245, D-46462 Wesel, Deutschland) oder Tego Twin 4000 (Fa. Tego Chemie Service GmbH, Goldschmidtstr. 100, D-451127 Essen, Deutschland) oder Surfynol 104, Surfynol 104PA oder Surfynol 504 (Fa. Air Products Chemicals Europe B.V., Kanaalweg 15, P.O. Box 3193, NL 3502 GD Utrecht, Niederlande).

Die Netzmittel werden in ihrer kommerziell erhältlichen Form eingesetzt. Dies sind meist Lösungen des Netzmittels in Lösemitteln. In Bezug auf den Anteil des Netzmittels an den erfindungsgemäßen Pigmentzusammensetzungen werden die Anteile des Netzmittels ohne den Lösemittelanteil verstanden.

Viele bekannte Pigmentpräparationen enthalten mit Wasser kompatible Bindemittel. Diese Bindemittel sind gut durch Wasser wieder anlösbar. Druckfilme, die aus derartigen Pigmentpräparationen hergestellt werden können, weisen durch den Einbau der aus der Pigmentpräparation stammenden, mit Wasser kompatiblen Bindemittel in den Farbfilm folglich eine schlechte Wasserbeständigkeit auf. Pigmentpräparationen, die Bindemittel enthalten, welche eine gute Kompatibilität zu organischen Lösemitteln aufweisen, zeigen hingegen eine eingeschränkte Kompatibilität zu wässrigen Applikationssystemen.

Aus diesen Gründen ist die erfindungsgemäße Pigmentzusammensetzung im Wesentlichen bindemittelfrei. Unter im Wesentliche bindemittelfrei wird vorzugsweise ein Anteil von weniger als 1 Gew.-%, weiter bevorzugt von unter 0,5 Gew.-%, noch weiter bevorzugt von unter 0,3 Gew.-%, besonders bevorzugt von unter 0,1 Gew.-%, bezogen auf die gesamte Pigmentzusammensetzung, verstanden. Ganz besonders bevorzugt enthält die Pigmentzusammensetzung überhaupt kein Bindemittel.

In den angegebenen geringen Mengen führen Bindemittel zu keiner bzw. keiner wesentlichen Beeinträchtigung bei der Kompatibilität der erfindungsgemäßen Pigmentzusammensetzung mit dem Applikationssystem und der Wasserbeständigkeit der Applikation, beispielsweise eines Druckerzeugnisses oder eines lackierten Gegenstandes.

Bindemittel, die in sehr geringen Mengen verwendet werden können sind bevorzugt solche, die für Druckfarben verwendet werden können wie beispielsweise Acrylate, Nitrocellulose, Polyvinylbutadiene, Cellulosebutyrate, Celluloseacetate, Polyurethane, Sulfopolyester, Alkydharze, Polyamide, Polyester, Melaminharze oder Maleinate.

Bei einer bevorzugten Ausführungsform enthält die erfindungsgemäße Pigmentzusammensetzung noch mindestens einen Entschäumer. Hierdurch ist eine bessere Einarbeitbarkeit vor allem in wässrige Systeme ohne Luftblasenbildung gegeben. Der Entschäumer wird in Mengen von 1 bis 10 Gew.-%, bevorzugt von 2 bis 7 Gew.- und besonders bevorzugt von 3 bis 5 Gew.-% bezogen auf die gesamte Pigmentzusammensetzung eingesetzt. Unterhalb von 1 Gew.-% ist seine Wirksamkeit zu gering, während oberhalb von 10 Gew.-% keine vorteilhaften Wirkungen mehr zu bemerken sind.

Als Entschäumer sind prinzipiell alle üblichen Entschäumersysteme geeignet. Dies sind beispielsweise Siliconöle, medizinische Weißöle oder pflanzliche Öle.

Kommerziell erhältliche Entschäumer sind beispielsweise BYK-071, BYK-A 530, BYK-060 N, BYK-018, BYK-044, BYK-020, BYK-065, BYK-066N oder BYK-067 der Fa. Byk-Chemie. Ebenfalls sind Verbindungen der Produktreihe Agitan® der Fa. Münzing Chemie GmbH (Salzstraße 174, D-74076 Heilbronn, Deutschland) geeignet.

Weiterhin geeignet sind Entschäumer auf Basis von Mischungen von Mineralölen. Kommerziell erhältliche Beispiele hierfür sind:
BYK-031, BYK-035, BYK-037, BYK-038 BYK-045 der Firma Byk-Chemie, Dapro DF 900, Dapro DF 911, Dapro DF 1492 der Firma Elementis Specialities p/a Elementis Service Centre N.V., Pegasus Park, De Kleetlaan 12A - P.O. Box 3, B-1831 Diegem, Belgien.

Als Lösemittel werden mit Wasser kompatible Lösemittel und deren Gemische verwendet. Hierunter fallen beispielsweise Alkohole, Glykole, Ketone, Aldehyde oder Ester.

Bevorzugt jedoch wird Wasser oder ein Wasser enthaltendes Lösemittelgemisch verwendet. Bei Verwendung eines Lösemittelgemischs ist bevorzugt, dass dieses im wesentlichen aus Wasser besteht. Vorzugsweise enthält ein solches Lösemittelgemisch wenigstens 70 Vol.-% Wasser, weiter bevorzugt wenigstens 80 Vol.-%, noch weiter bevorzugt wenigstens 90 Vol.-%. Gemäß einer weiteren Variante enthält das Lösemittelgemisch wenigstens 95 Vol.% Wasser.

Ein Verfahren zur Herstellung der erfindungsgemäßen Pigmentzusammensetzung besteht daraus, dass die einzelnen Komponenten, vorzugsweise schonend, miteinander homogen vermischt werden. Die Vermischung kann in kommerziell üblichen Mischern, Knetern oder einem Rollbock vonstatten gehen. Im Prinzip können die einzelnen Komponenten der erfindungsgemäßen Pigmentzusammensetzung in beliebiger Reihenfolge in das Mischaggregat aufgegeben und dann miteinander unter möglichst schonenden Bedingungen miteinander vermischt werden. Eine schonende Behandlung ist bevorzugt, da die scherempfindlichen Perlglanzpigmente ansonsten zerstört werden könnten.

Bei einem bevorzugten Verfahren werden zunächst das Netzmittel und ggfs. der Entschäumer im Lösemittel gelöst und diese Lösung/Dispersion mit dem Perlglanzpigment miteinander im Mischaggregat vermischt. Dieses Verfahren gewährleistet eine optimale Homogenität der Pigmentzusammensetzung.

Die erfindungsgemäßen Pigmentzusammensetzungen finden Verwendung in Druckfarben, Lacken und kosmetischen Formulierungen. Als Druckfarben kommen alle bekannten Druckfarben wie Offset-, Flexo-, Sieb- und Tiefdruckfarben in Frage. Bevorzugt wird die erfindungsgemäße Pigmentzusammensetzung in wässrigen Druckfarben und wässrigen Lacken verwendet.

Wässrige Druckfarben finden wiederum Verwendung bei Tapeten, Faltschachteln, Geschenkpapier oder Tissues. Konventionelle Druckfarben werden beispielsweise in der flexiblen Folienverpackung oder im Etikettendruck eingesetzt.
Die folgenden Beispiele erläutern die Erfindung, ohne sie jedoch zu beschränken.

### Beispiele

### Beispiel 1:

5 g Surfynol 104 (Netzmittel, Fa. Air Products) sowie 2 g Agitan 760 (Entschäumer) werden unter Rühren in 18 g Wasser gelöst. Diese Lösung wird mit 75 g PHOENIX PX 2031 (kommerziell erhältliches Perlglanzpulver, Fa. Eckart) in einem Butterflymischer vermischt. Man erhält ein nichtstaubendes Perlglanzpulver.

### Beispiel 2:

8 g Surfynol 504 (Netzmittel, Fa. Air Products) sowie 5 g Dapro DF 900 (Entschäumer) werden unter Rühren in 17 g Wasser gelöst. Diese Lösung wird mit 70 g PHOENIX PX 3001 (kommerziell erhältliches Perlglanzpulver, Fa. Eckart) in einem Butterflymischer vermischt. Man erhält ein nichtstaubendes Perlglanzpulver.

### Beispiel 3:

5 g Surfynol 104PA (Netzmittel, Fa. Air Products) sowie 5 g Byk 052 (Entschäumer) werden unter Rühren in 20 g Isopropanol gelöst. Diese Lösung wird mit 70 g PHOENIX PX 1000 (kommerziell erhältliches Perlglanzpulver, Fa. Eckart) in einem Butterflymischer vermischt. Man erhält ein nichtstaubendes Perlglanzpulver.

### Beispiel 4:

2 g Tego Twin 4000 (Netzmittel, Fa. Tego) sowie 2 g No Foam (Entschäumer) werden unter Rühren in 16 g Wasser gelöst. Diese Lösung wird mit 80 g Iriodin 123 (kommerziell erhältliches Perlglanzpulver, Fa. Merck) in einem Butterflymischer vermischt. Man erhält ein nichtstaubendes Perlglanzpulver.

### Vergleichsbeispiel 5:

5 g Surfynol 104 (Netzmittel, Fa. Air Products) sowie 2 g Agitan 760 (Entschäumer) werden unter Rühren in 18 g Wasser gelöst. Diese Lösung wird mit 50 g PHOENIX PX 2031 (kommerziell erhältliches Perlglanzpulver, Fa. Eckart) in einem Butterflymischer vermischt. Man erhält statt eines Pulvers eine dilatante Paste.

### Vergleichsbeispiel 6:

5 Hydropalat 88 (anionisches Netzmittel auf Basis eines Sulfobernsteinsäureesters, Fa. Henkel) sowie 2 g Agitan 760 (Entschäumer) werden unter Rühren in 22 g Wasser gelöst. Diese Lösung wird mit 70 g PHOENIX PX 3001 (kommerziell erhältliches Perlglanzpulver, Fa. Eckart) in einem Butterflymischer vermischt. Man erhält ein nichtstaubendes Perlglanzpulver

### Vergleichsbeispiel 7:

2 g Byk 333 (Netzmittel auf Basis eines Polyethermodifizierten Dimethylpolysiloxans ohne OH-Funktionen, Fa. Byk) sowie 2 g No Foam (Entschäumer) werden unter Rühren in 16 g Wasser gelöst. Diese Lösung wird mit 80 g Iriodin 123 (kommerziell erhältliches Perlglanzpulver, Fa. Merck) in einem Butterflymischer vermischt. Man erhält ein nichtstaubendes Perlglanzpulver.

### Vergleichsbeispiel 8:

15 g Luviskol K 17 (Polyvinylpyrrolidon-Harz, Fa. BASF) und 5 g Surfynol 104 werden unter Rühren in 20 g Wasser gelöst. Diese Lösung wird mit 80 g PHOENIX PX 2301 (kommerziell erhältliches Perlglanzpulver, Fa. Eckart) in einem Butterflymischer vermischt. Man erhält ein nichtstaubendes Perlglanzpulver.

Die Abprüfung der Pigmentzusammensetzungen gemäß den oben aufgeführten erfindungsgemäßen Beispielen und Vergleichsbeispielen erfolgte jeweils in Druckapplikationen. Es wurde jeweils eine Flexodruckfarbe auf Basis eine kommerziell erhältlichen Acrylatsystems hergestellt. Die Pigmentzusammensetzungen der Beispiele wurden so dosiert, dass eine Konzentration von 26 Gew.-% Perlganzpigment, bezogen auf die Gesamt-Druckfarbe, eingestellt wurde. Dabei wurden die Druckfarben mit folgenden Aggregaten appliziert:
1) Labor-Tiefdruck: Laborandruck mittels K 303 Multicoater (Fa. Saueressig, Deutschland)
2) Labor-Flexodruck: Laborandrucke mittels Flexiproof 100 (Fa. Erichsen, Deutschland)
3) Tief-/Flexo-Druckmaschine: produktionsrelevante Andrucke mittels Tief-/Flexodruckmaschine ROTOVA 300 (Fa. RotoColor, Schweiz), Geschwindigkeit 150 m/min.

Die Überprüfung der Druckfilmeigenschaften (Wasserbeständigkeit) erfolgte immer erst nach kompletter Trocknung des Farbfilms.

Die Laborapplikationen (1,2) wurden über Nacht bei Raumtemperatur getrocknet, während die Trocknung der Applikationen auf der Tief-/Flexodruckmaschine bei 80 °C unter einem Heißluftstrom bei gegebener Bandgeschwindigkeit erfolgte.

Die Wasserfestigkeit wurde gemäß der DIN 16 524 Blatt 1 überprüft.

Es wurden keine wesentlichen Unterschiede der Eigenschaften der Drucke der unterschiedlichen Applikationssysteme festgestellt.

**Tab. 1**

| **Probe** | **Probenkonsistenz** | **Qualität Film** | **Wasserbeständigkeit** |
|---|---|---|---|
| Beispiel 1 | nichtstaubendes Perlglanzpulver | sehr gut | Erfüllt Kriterien der DIN 16 524 Blatt 1 |
| Beispiel 2 | nichtstaubendes Perlglanzpulver | sehr gut | " |
| Beispiel 3 | nichtstaubendes Perlglanzpulver | sehr gut | " |
| Beispiel 4 | nichtstaubendes Perlglanzpulver | sehr gut | " |
| Vergleichs-Beispiel 5 | dilatante Paste | sehr gut | " |
| Vergleichs-Beispiel 6 | nichtstaubendes Perlglanzpulver | Oberflächendefekte, (starke Schaumbildung) | " |
| Vergleichs-Beispiel 7 | nichtstaubendes Perlglanzpulver | Oberflächen-Störungen; reduzierte Brillanz | " |
| Vergleichs-Beispiel 8 | nichtstaubendes Perlglanzpulver | sehr gut | Sehr schlechte Wasserbeständigkeit |

Tabelle 1 ist zu entnehmen, das die erfindungsgemäßen Beispiele 1 bis 4 nichtstaubende Perlglanzpigmentpulver ergeben. Diese nichtstaubenden

Pigmentpulver lassen sich sehr leicht in Druckfarben einarbeiten. Die unter Verwendung dieser Druckfarben hergestellten Druckfilme weisen eine sehr gute Qualität auf und sind wasserbeständig.

Die gemäß dem Vergleichsbeispiel 5 erhaltene Pigmentzusammensetzung hat eine niedrige Perlglanzpigmentkonzentration von nur 50 Gew.-%. Hier entsteht keine pulverförmige Pigmentzusammensetzung, sondern vielmehr eine Pigmentpaste mit dilatanten viskosen Eigenschaften, was nicht erwünscht ist. Die Einarbeitung dieser Pigmentzusammensetzung in die Druckfarbe gestaltete sich extrem schwierig. Aufgrund dieser schweren Einarbeitbarkeit ist eine solche Pigmentzusammensetzung in der Praxis nicht verwendbar.

Bei der Herstellung der Pigmentzusammensetzung gemäß Vergleichsbeispiel 6 wurde ein anionisches Netzmittel verwendet. Trotz Zugabe eines Entschäumers kam es zu unerwünschter starker Schaumentwicklung bei der Einarbeitung dieser Pigmentzusammensetzung in die Druckfarbe. Ebenfalls wurden Oberflächendefekte des Druckfilmes beobachtet, die auf eine ungenügende Pigmentbenetzung zurückzuführen sind.

Eine sehr schlechte Wasserbeständigkeit wurde bei der Pigmentzusammensetzung gemäß Vergleichsbeispiel 8, bei dem anstelle des nichtionischen Netzmittels ein wasserlösliches Harz verwendet wurde, erhalten.

## Patentansprüche

1. Nichtstaubende pulverförmige, im wesentlichen bindemittelfreie Pigmentzusammensetzung,
**dadurch gekennzeichnet,**
**dass** die Pigmentzusammensetzung folgende Bestandteile enthält:
- wenigstens 60 Ges.-% mindestens eines Perlglanzpigmentes
- 1 -15 Gew.-% nichtionisches monomeres Netzmittel und/oder nichtionisches Netzmittel auf Polysiloxan-Basis
- 1 bis 39 Gew.-% Lösemittel oder Lösemittelgemisch,
wobei sich die Summe der Einzelkomponenten zu 100 Gew.-% addiert, wobei
das nichtionische Netzmittel OH-Gruppen trägt und
eine Hydroxylzahl von 30 bis 150 mg KOH/g Netzmittel, vorzugsweise von 50 bis 120 mg KOH/g Netzmittel, aufweist.

2. Nichtstaubende pulverförmige Pigmentzusammensetzung nach Anspruch 1,
**dadurch gekennzeichnet,**
**dass** die Pigmentzusammensetzung zusätzlich 1 bis 10 Gew.-%; vorzugsweise 2 bis 7 Gew.-%, Entschäumer enthält.

3. Nichtstaubende pulverförmige Pigmentzusammensetzung nach einem der vorstehenden Ansprüche,
**dadurch gekennzeichnet,**
**dass** die Perlglanzpigmente aus der Gruppe, die aus mit hochbrechendem/en Metalloxid(en) beschichteten Glimmer, mit hochbrechendem/en Metalioxid(en) beschichteten Glasplättchen, mit hochbrechendem/en Metalloxid(en) beschichteten SiO₂-Flakes, mit hochbrechendem/en Metalioxid(en) beschichteten Al₂O₃-Flakes, TiO₂-Flakes, Eisenoxidflakes und deren Mischungen, besteht, ausgewählt wird.

4. Nichtstaubende pulverförmige Pigmentzusammensetzung nach einem der vorstehenden Ansprüche,
**dadurch gekennzeichnet,**
**dass** das Lösemittel Wasser ist oder dass das Lösemittelgemisch im wesentlichen aus Wasser besteht.

5. Nichtstaubende pulverförmige Pigmentzusammensetzung nach einem der vorstehenden Ansprüche,
**dadurch gekennzeichnet,**
**daß** die Pigmehtzüsammensetzung weniger als 1 Gew.-%, vorzugsweise weniger als 0,5 Gew.-%, Bindemittel enthält.

6. Nichtstaubende pulverförmige Pigmentzusammensetzung nach einem der vorstehenden Ansprüche,
**dadurch gekennzeichnet,**
**daß** die Pigmentzusammensetzung bindemittelfrei ist.

7. Nichtstaubende pulverförmige Pigmentzusammensetzung nach einem der vorstehenden Ansprüche,
**dadurch gekennzeichnet,**
**daß** das monomere Netzmittel folgende Struktur (I) aufweist:

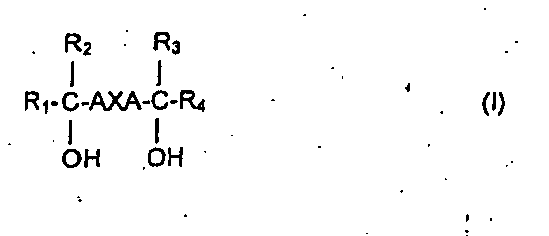

8. Nichtstaubende pulverförmige Pigmentzusammensetzung nach Anspruch 7,
**dadurch gekennzeichnet,**
**daß** R₁, R₂,R₃ und R₄ unabhängig voneinander Methyl, Ethyl, n-Propyl, Isopropyl, tert-Butyl sind.

9. Nichtstaubende pulverförmige Pigmentzusammensetzung nach Anspruch 7 oder 8,
**dadurch gekennzeichnet,**
**daß** R₁, und R₄ jeweils tert-Butyl und R₂ und R₃ vorzugsweise jeweils Methyl sind.

10. Verfahren zur Herstellung einer nichtstaubenden pulverförmigen, im wesentlichen bindemittelfreien Pigmentzusammensetzung nach einem der Ansprüche 1 bis 9, **dadurch gekennzeichnet,**
**dass** die einzelnen Komponenten, vorzugsweise schonend, miteinander homogen vermischt werden.

11. Verwendung der nichtstaubenden pulverförmigen, im Wesentlichen bindemittelfreien Pigmentzusammensetzung nach einem der Ansprüche 1 bis 9 in Druckfarben. Lacken und kosmetischen Formulierungen.

## Claims

1. Dust-free, powdered pigment composition essentially free of binding agent,
**characterised in that**
the pigment composition contains the following elements:
- at least 60 % by weight of at least one nacreous pigment,
- 1 - 15 % by weight of non-ionic monomeric wetting agent and/or non-ionic wetting agent with a polysiloxane base,
1 to 39 % by weight of solvent or solvent mixture,
the sum of the individual components adding up to 100 % by weight, and the non-ionic wetting agent contains OH groups and the wetting agent has a hydroxyl number of 30 to 150 mg KOH/g, preferably 50 to 120 mg KOH/g.

2. Dust-free, powdered pigment composition as claimed in claim 1,
**characterised in that**
the pigment composition additionally contains 1 to 10 % by weight, preferably 2 to 7 % by weight, of defoaming agent.

3. Dust-free, powdered pigment composition as claimed in one of the preceding claims,
**characterised in that**
the nacreous pigments are selected from the group comprising mica coated with highly refractive metal oxide(s), glass platelets coated with highly refractive metal oxide(s), SiO₂ flakes coated with highly refractive metal oxide(s), Al₂O₃ flakes coated with highly refractive metal oxide(s), TiO₂ flakes, iron oxide flakes and mixtures thereof.

4. Dust-free, powdered pigment composition as claimed in one of the preceding claims,
**characterised in that**
the solvent is water or the solvent mixture essentially comprises water.

5. Dust-free, powdered pigment composition as claimed in one of the preceding claims,
**characterised in that**
the pigment composition contains less than 1 % by weight, preferably less than 0.5 % by weight, of binding agent.

6. Dust-free, powdered pigment composition as claimed in one of the preceding claims,
**characterised in that**
the pigment composition is free of binding agent.

7. Dust-free, powdered pigment composition as claimed in one of the preceding claims,
**characterised in that**
the monomeric wetting agent has the following structure (I): 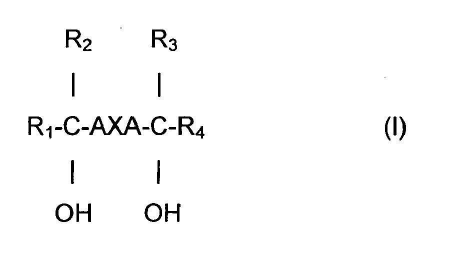 where R₁, R₂, R₃ and R₄ independently of one another stand for hydrogen, an alkyl residue with a straight chain or branched chain with 1 to 10 carbon atoms respectively, each of which may contain OH groups, an aryl residue with 1 to 12 carbon atoms, and the aryl residue may be substituted with alkyl residues with 1 to 10 carbon atoms, and the alkyl and/or aryl residue may each contain OH groups, X stands for a single bond, double bond or triple bond, A stands for CH₂ if X is a single bond, A stands for CH if X is a double bond and A stands for C if X is a triple bond.

8. Dust-free, powdered pigment composition as claimed in claim 7,
**characterised in that**
R₁, R₂, R₃ and R₄, independently of one another, are methyl, ethyl, n-propyl, isopropyl, n-butyl, isobutyl, tert-butyl.

9. Dust-free, powdered pigment composition as claimed in claim 7 or 8,
**characterised in that**
R₁ and R₄ are each tert-butyl and R₂ and R₃ are preferably each methyl.

10. Method of producing a dust-free, powdered pigment composition essentially free of binding agent as claimed in one of claims 1 to 9,
**characterised in that**
the individual components are homogenously mixed with one another, preferably gently.

11. Use of the dust-free, powdered pigment composition essentially free
of binding agent as claimed in one of claims 1 to 9 in printing inks, varnishes and cosmetic formulations.

## Revendications

1. Composition de pigments sous forme de poudre ne dégageant pas de poussières, sensiblement dépourvue de liants,
**caractérisée en ce**
**que** la composition de pigments comprend les composants suivants :
- au moins 60 % en poids d'au moins un pigment nacré
- 1 à 15 % en poids d'un agent mouillant monomère non ionique et/ou d'un agent non ionique à base de polysiloxane
- 1 à 39 % en poids de solvant ou de mélange de solvants,
la somme des composants individuels constituant 100 % en poids, où
l'agent mouillant non ionique porte des groupes OH et présente un indice d'hydroxyle de 30 à 150 mg de KOH/g d'agent mouillant, de préférence de 50 à 120 mg de KOH/g d'agent mouillant.

2. Composition de pigment sous forme de poudre ne dégageant pas de poussières selon la revendication 1,
**caractérisée en ce**
**que** la composition de pigment comprend en plus 1 à 10 % en poids, de préférence 2 à 7 % en poids d'agents antimousses.

3. Composition de pigment sous forme de poudre ne dégageant pas de poussières selon l'une des revendications précédentes,
**caractérisée en ce**
**que** les pigments nacrés sont choisis dans le groupe constitué par le mica revêtu d'un ou de plusieurs oxydes métalliques à indice de réfraction élevé, les lamelles de verre revêtues d'un ou de plusieurs oxydes métalliques à indice de réfraction élevé, les paillettes de SiO₂ revêtues d'un ou de plusieurs oxydes métalliques à indice de réfraction élevé, les paillettes de Al₂O₃ revêtues d'un ou de plusieurs oxydes métalliques à indice de réfraction élevé, les paillettes de TiO₂, les paillettes d'oxyde de fer et leurs mélanges.

4. Composition de pigment sous forme de poudre ne dégageant pas de poussières selon l'une des revendications précédentes,
**caractérisée en ce**
**que** le solvant est l'eau ou en ce que le mélange de solvants se compose essentiellement d'eau.

5. Composition de pigment sous forme de poudre ne dégageant pas de poussières selon l'une des revendications précédentes,
**caractérisée en ce**
**que** la composition de pigment contient moins de 1 % en poids, de préférence moins de 0,5 % en poids de liant.

6. Composition de pigment sous forme de poudre ne dégageant pas de poussières selon l'une des revendications précédentes,
**caractérisée en ce**
**que** la composition de pigments est dépourvue de liants.

7. Composition de pigment sous forme de poudre ne dégageant pas de poussières selon l'une des revendications précédentes,
**caractérisée en ce**
**que** l'agent mouillant monomère présente la structure suivante (I) : 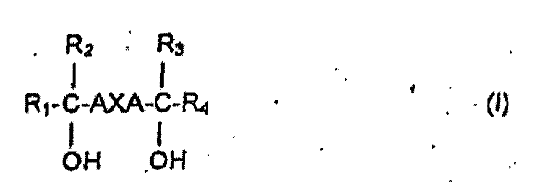 sachant que R₁, R₂, R₃ et R₄ peuvent être, indépendamment l'un de l'autre, hydrogène, un radical alkyle linéaire ou ramifié ayant respectivement 1 à 10 atomes de carbone, qui peut présenter respectivement des groupes OH, un radical aryle ayant de 1 à 12 atomes de carbone, où le radical aryle peut être substitué par des radicaux alkyles ayant de 1 à 10 atomes de carbone, où le radical alkyle et/ou aryle peuvent présenter respectivement des groupes OH, et X représente une simple liaison, une double liaison ou une triple liaison, où A représente CH₂, quand X est une simple liaison, A représente CH, quand X est une double liaison, et A représente C quand X est une triple liaison.

8. Composition de pigment sous forme de poudre ne dégageant pas de poussières selon la revendication 7,
**caractérisée en ce**
**que** R₁, R₂, R₃ et R₄ sont, indépendamment l'un de l'autre, méthyle, éthyle, n-propyle, isopropyle, n-butyle, isobutyle, tert.-butyle.

9. Composition de pigment sous forme de poudre ne dégageant pas de poussières selon la revendication 7 ou 8,
**caractérisée en ce**
**que** R₁ et R₄ sont respectivement tert.-butyle et R₂ et R₃ sont de préférence respectivement méthyle.

10. Procédé de fabrication d'une composition de pigment sous forme de poudre ne dégageant pas de poussières, sensiblement dépourvue de liants, selon l'une des revendications 1 à 9,
**caractérisé en ce**
**que** les différents composants sont mélangés ensemble de manière homogène, de préférence doucement.

11. Utilisation de la composition de pigment sous forme de poudre ne dégageant pas de poussières, sensiblement dépourvue de liants, selon l'une des revendications 1 à 9 dans des encres d'impression, des laques et des formulations cosmétiques.
